Europäisches Patentamt

European Patent Office

Office européen des brevets

(19)

(11) EP 0 898 976 B1

(12) **FASCICULE DE BREVET EUROPEEN**

(45) Date de publication et mention
de la délivrance du brevet:
**03.12.2003 Bulletin 2003/49**

(51) Int Cl.⁷: **A61M 1/16**

(21) Numéro de dépôt: **98420144.2**

(22) Date de dépôt: **07.08.1998**

(54) **Appareil de dialyse permettant de contrôler, de façon indépendante, la concentration d'au moins deux substances ioniques dans le milieu intérieur d'un patient**

Dialysegerät mit unabhängiger Kontrolle von mindestens zwei ionischen Substanzen in dem Körper eines Patienten

Dialysis apparatus with independent control of at least two ionic substances in the patient's body

(84) Etats contractants désignés:
**AT BE CH DE ES FR GB IT LI NL SE**

(30) Priorité: **21.08.1997 FR 9710670**

(43) Date de publication de la demande:
**03.03.1999 Bulletin 1999/09**

(73) Titulaire: **HOSPAL INDUSTRIE**
**F-69883 Meyzieu Cédex (FR)**

(72) Inventeurs:
• **Bene, Bernard**
**69540 Irigny (FR)**

• **Burtin, Jacques**
**69320 Feyzin (FR)**

(74) Mandataire: **Sutto, Luca et al**
**Gambro Intellectual Property Department**
**Hospal International Marketing Management**
**61, avenue Tony Garnier**
**69007 Lyon (FR)**

(56) Documents cités:
**EP-A- 0 532 433          DE-A- 4 114 908**

**Description**

La présente invention a pour objet appareil de dialyse permettant de contrôler, de façon indépendante, la concentration d'au moins deux substances ioniques dans le milieu intérieur d'un patient.

Les reins remplissent de multiples fonctions parmi lesquelles l'élimination d'eau, l'excrétion des catabolites (ou déchets du métabolisme, tels que l'urée, la créatinine), la régulation de la concentration des électrolytes du sang (sodium, potassium, magnésium, calcium, bicarbonates, phosphates, chlorures) et la régulation de l'équilibre acido-basique du milieu intérieur qui est obtenue notamment par l'élimination des acides faibles (phosphates, acides monosodiques) et par la production de sels d'ammonium.

Chez les personnes qui ont perdu l'usage de leurs reins, ces mécanismes excréteurs et régulateurs ne jouant plus, le milieu intérieur se charge en eau et en déchets du métabolisme et présente un excès d'électrolytes (de sodium en particulier), ainsi que, en général, une acidose, le pH du plasma sanguin se déplaçant vers 7 (le pH sanguin varie normalement dans des limites étroites comprises entre 7,35 et 7,45).

Pour pallier le dysfonctionnement des reins, on recourt classiquement à un traitement du sang par circulation extracorporelle dans un échangeur à membrane semi-perméable (hémodialyseur) où l'on fait circuler, d'un côté et de l'autre de la membrane, le sang du patient et un liquide de dialyse comprenant les principaux électrolytes du sang, dans des concentrations proches de celles du sang d'un sujet sain. Par l'effet du phénomène physique appelé dialyse, les molécules migrent du liquide où leur concentration est la plus élevée vers le liquide où leur concentration est la moins élevée.

Dans un appareil de dialyse classique, le liquide de dialyse est préparé par un mélange dosé d'eau et de deux solutions concentrées, une première solution concentrée contenant du chlorure de sodium et du bicarbonate de sodium et la seconde solution concentrée contenant des chlorures de calcium, de potassium et de magnésium ainsi que de l'acide acétique. L'acide acétique a pour fonction de limiter la formation de précipités de carbonate de calcium et de magnésium qui forment des dépôts indésirables dans le circuit hydraulique de l'appareil de dialyse.

Ce mode de préparation classique d'un liquide de dialyse présente plusieurs inconvénients:

- les concentrations respectives des différentes substances ioniques entrant dans la composition du liquide de dialyse ne peuvent pas être réglées indépendamment les unes des autres, alors que cela serait souhaitable au moins pour le sodium, le potassium et le bicarbonate;
- pour des raisons physiologiques, la concentration de l'acide acétique dans le liquide de dialyse est nécessairement limitée, de sorte qu'il se forme dans le circuit hydraulique des appareils de dialyse des dépôts carbonatés. Les appareils de dialyse doivent donc être détartrés régulièrement, ce qui en alourdit la maintenance;
- la présence d'acide acétique dans la deuxième solution provoque la corrosion des moyens de connexion et de pompage utilisés pour transférer la solution concentrée du réservoir où elle est contenue jusqu'à la zone de mélange de l'appareil où elle est diluée dans la solution obtenue par mélange dosé d'eau et de la première solution;
- l'utilisation d'un liquide de dialyse ayant un pH inférieur à celui du sang au moment de l'amorçage de certains types d'hémodialyseurs, c'est-à-dire au moment où le compartiment de liquide de dialyse de ces hémodialyseurs est rempli de liquide de dialyse et le compartiment sang est rempli de sang dilué, semble être un des cofacteurs de certaines réactions d'hypersensibilité.

Le document EP 0 192 588 décrit un appareil de dialyse comprenant:

- des moyens pour mettre en circulation un liquide de dialyse contenant du bicarbonate de sodium et dépourvu de calcium et de magnésium,
- des moyens pour perfuser au patient une solution de perfusion contenant au moins du calcium et du magnésium.

Avec cet appareil, comme les substances ioniques (bicarbonate, calcium, magnésium) susceptibles de former des précipités quand elles sont mises en présence, sont séparées, il n'est pas nécessaire de faire entrer de l'acide acétique dans la composition du liquide de dialyse. Tel qu'il est décrit, cet appareil est cependant inutilisable car il ne comprend pas de moyens de réglage du débit de perfusion qui tiendrait compte en particulier du transfert diffusif de calcium et de magnésium au travers de la membrane du dialyseur, du sang du patient vers le liquide de dialyse. Or pour des raisons de sécurité, il n'est pas envisageable de perfuser à un patient une solution concentrée de calcium sans pouvoir régler précisément le débit de perfusion de façon que le milieu intérieur du patient ne soit le lieu ni d'un excès ni d'un déficit de cette substance ionique.

Le but de l'invention est de réaliser un appareil de dialyse permettant de régler précisément le débit d'un liquide de perfusion contenant une substance ionique en vue de faire tendre vers une concentration souhaitée la concentration de cette substance dans le milieu intérieur du patient. Plus largement le but de l'invention est de réaliser un appareil de dialyse permettant d'agir séparément sur la concentration d'au moins deux substances ioniques dans le milieu intérieur d'un patient au moyen d'un liquide de dialyse et d'un liquide de perfusion.

Ce but est atteint au moyen d'un appareil de dialyse comprenant :

- des moyens pour mettre en circulation dans un hémodialyseur un liquide de dialyse contenant du chlorure de sodium et du bicarbonate de sodium;
- des moyens pour perfuser à un patient au moins une solution contenant au moins une substance ionique A (calcium, magnésium, potassium) absente du liquide de dialyse, la substance A ayant une concentration déterminée [A]sol, dans la solution de perfusion;

   caractérisé en ce qu'il comporte :

- des moyens pour déterminer la dialysance réelle D de l'hémodialyseur pour le sodium;
- des moyens pour déterminer un débit Qinf de solution de perfusion pour que la concentration de la substance A dans le milieu intérieur du patient tende vers une concentration souhaitée [A]des, en fonction de la dialysance D, de la concentration [A]sol de la substance A dans la solution de perfusion, et de la concentration souhaitée [A]des;
- des moyens de réglage pour régler le débit de solution de perfusion;
- des moyens de commande pour piloter les moyens de réglage du débit de la solution de perfusion pour que ce débit soit sensiblement égal au débit déterminé Qinf.

Dans l'appareil de dialyse selon l'invention, le liquide de dialyse étant dépourvu de calcium, de magnésium et, éventuellement, de potassium, ces substances, qui sont présentes dans le sang, migrent par diffusion, au cours de la séance de dialyse, du sang vers le liquide de dialyse. C'est pour compenser ces pertes diffusives que l'on prévoit de perfuser au patient une solution contenant ces substances. La difficulté réside en ce que ces pertes diffusives sont variables au cours d'une séance de dialyse de plusieurs heures et en ce que l'excès comme le déficit de potassium, de calcium et de magnésium dans le sang du patient peut engendrer des troubles graves, notamment des troubles cardiaques. Grâce à l'invention cette difficulté est surmontée, puisque le débit de la perfusion est asservi à la dialysance réelle du système de traitement.

Par ailleurs, outre de remédier aux inconvénients des appareils de dialyse classiques mentionnés plus haut, l'appareil selon l'invention présente aussi l'avantage suivant : pourvu que le liquide de perfusion soit injecté directement dans le patient ou, en aval de l'hémodialyseur, dans le circuit de circulation extracorporelle de sang reliant le patient à l'hémodialyseur, le calcium ionique du sang migre par diffusion dans le liquide de dialyse puisque celui-ci est dépourvu de calcium. Or le calcium ionique intervient dans la cascade de réactions constituant le processus de coagulation du sang. L'appauvrissement massif du sang en calcium dans l'hémodialyseur inhibe donc partiellement le processus de coagulation, ce qui permet de réduire la quantité d'anticoagulant injectée usuellement dans le circuit de circulation extracorporelle de sang pour y prévenir la coagulation du sang.

Selon une caractéristique de l'invention, les moyens de détermination du débit de perfusion Qinf comprennent des moyens de calcul pour calculer le débit de perfusion Qinf selon la formule :

$$Qinf = Cl \times \frac{[A]des}{[A]sol - [A]des}$$

où Cl est la clairance de l'hémodialyseur pour la substance A, extrapolée à partir de la dialysance D pour le sodium.

Selon une autre caractéristique de l'invention, la solution de perfusion contient du sodium à une concentration déterminée [Na+]sol. Dans ce cas l'appareil comporte en outre:

- des moyens de préparation du liquide de dialyse comprenant des moyens pour régler la concentration en sodium du liquide de dialyse;
- des moyens pour déterminer la concentration en sodium [Na+]dial du liquide de dialyse pour que la concentration du milieu intérieur du patient tende vers une concentration souhaitée en sodium [Na+]des, en fonction de la dialysance D, du débit du liquide de perfusion Qinf, de la concentration en sodium [Na+]sol de la solution de perfusion et de la concentration en sodium souhaitée [Na+]des;
- des moyens de commande pour piloter les moyens de réglage de la concentration en sodium du liquide de dialyse de façon que cette concentration soit égale à la concentration déterminée [Na+]dial.

Selon une caractéristique de l'invention, les moyens pour déterminer la concentration en sodium [Na+]dial du liquide de dialyse comprennent des moyens de calcul pour calculer cette concentration selon la formule :

**EP 0 898 976 B1**

$$[Na^+]dial = \frac{Qinf}{D} ([Na^+]des - [Na^+]sol) + [Na^+]des$$

D'autres caractéristiques et avantages de l'invention apparaîtront mieux à la lecture de la description qui va suivre. On se reportera à l'unique figure annexée qui représente de façon schématique un système d'hémodialyse selon l'invention.

Le système d'hémodialyse représenté sur la figure comprend un hémodialyseur 1 ayant deux compartiments 2, 3 séparés par une membrane semi-perméable 4. Un premier compartiment 2 a une entrée connectée à une canalisation 5 de prélèvement de sang sur laquelle est disposée une pompe de circulation 6, et une sortie connectée à une canalisation 7 de restitution de sang sur laquelle est interposé un piège à bulles 8.

Un dispositif de perfusion comprenant une pompe 10 et une balance 11 est prévu pour injecter dans le piège à bulles 8 le contenu d'une poche 9 de liquide de perfusion. La poche 9 est suspendue à la balance 11 et elle est reliée au piège à bulles 8 par une canalisation 12 sur laquelle est disposée la pompe de perfusion 10. La balance 11 sert à piloter la pompe 10 pour que le débit du liquide de perfusion soit égal à un débit de consigne.

Le second compartiment 3 de l'hémodialyseur 1 a une entrée connectée à une canalisation 12 d'alimentation en liquide de dialyse frais et une sortie connectée à une canalisation 13 d'évacuation de liquide usé (liquide de dialyse et ultrafiltrat).

La canalisation d'alimentation 12 relie l'hémodialyseur 1 à un dispositif de préparation de liquide de dialyse 14 comprenant une canalisation principale 15 à laquelle sont connectées en série deux canalisations de dérivation secondaires 16, 17. L'extrémité amont de la canalisation principale 15 est prévue pour être raccordée à une source d'eau courante. Chaque canalisation secondaire 16, 17 comprend des moyens de raccord pour le montage d'une cartouche 18, 19 contenant un sel sous forme de granulés. Une pompe 20, 21 est disposée sur chaque canalisation secondaire 16, 17 en aval de la cartouche 18, 19 correspondante pour y faire circuler le liquide provenant de la canalisation principale. Chaque pompe 20, 21 est pilotée en fonction de la comparaison entre 1) une valeur de consigne de conductivité pour le mélange de liquides se formant à la jonction de la ligne principale 15 et de l'extrémité aval de la ligne secondaire 16, 17 et 2) la valeur de la conductivité de ce mélange mesurée au moyen d'une sonde de conductivité 22, 23 disposée sur la canalisation principale 15 immédiatement en aval de la jonction entre la canalisation principale 15 et l'extrémité aval de la canalisation secondaire 16, 17.

La canalisation d'alimentation 12 forme le prolongement de la canalisation principale 15 du dispositif 14 de préparation de liquide de dialyse. Sur cette canalisation d'alimentation sont disposés, dans le sens de circulation du liquide, un premier débitmètre 24 et une première pompe de circulation 25.

L'extrémité aval de la canalisation 13 d'évacuation de liquide usé est prévue pour être reliée à l'égout. Sur cette canalisation sont disposés, dans le sens de circulation du liquide, une deuxième pompe de circulation 26 et un deuxième débitmètre 27. Une pompe d'extraction 28 est reliée à la canalisation d'évacuation 13, en amont de la deuxième pompe de circulation 26. La pompe d'extraction 28 est piloté de façon que son débit soit égal à une valeur de consigne de débit d'ultrafiltration dans l'hémodialyseur 1.

La canalisation d'alimentation 12 et la canalisation d'évacuation 13 sont reliées par une première et une seconde canalisations de dérivation 29, 30, qui sont reliées entre elles par une canalisation de jonction 31 sur laquelle est disposée une sonde de conductivité 32. Ces canalisations de dérivation 29, 30 et de jonction 31 et la sonde de conductivité 32 forment un dispositif de mesure de la conductivité du liquide de dialyse frais et usé qui est utilisé, comme cela sera expliqué plus loin, pour déterminer la dialysance (ou la clairance) réelle du système pour le sodium ou autre substance de masse moléculaire similaire. La première canalisation de dérivation 29 est reliée à la canalisation d'alimentation 12, en aval de la première pompe de circulation 25, par l'intermédiaire d'une première vanne trois voies 33, et elle est reliée à la canalisation d'évacuation 13, en amont de la deuxième pompe de circulation 26, par l'intermédiaire d'une deuxième vanne trois voies 34. La deuxième canalisation de dérivation 30 et la canalisation de jonction 31 sont reliées par l'intermédiaire d'une troisième vanne trois voies 35.

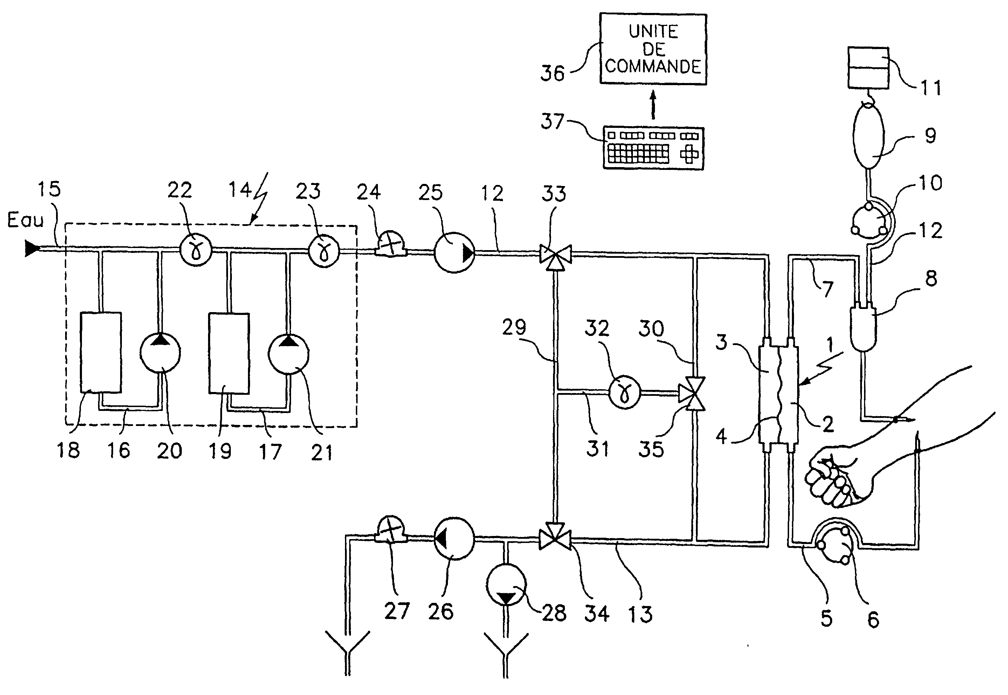

Le système d'hémodialyse représenté sur la figure 1 comprend également une unité de calcul et de commande 36. Cette unité est reliée à une interface utilisateur (clavier alphanumérique) 37 par laquelle elle reçoit des instructions, telles que diverses valeurs de consigne. Par ailleurs, elle reçoit des informations émises par les organes de mesure du système, tels que les débitmètres 24, 27, les sondes de conductivité 22, 23, 32 et la balance 11. Elle pilote, en fonction des instructions reçues et de modes d'opération et d'algorithmes programmés les organes moteurs du système, tels que les pompes 6, 10, 20, 21, 25, 26, 28 et les vannes 33, 34, 35.

Dans le mode de réalisation de l'invention représenté sur la figure,

- la cartouche 18 ne contient que du bicarbonate de sodium;
- la cartouche 19 ne contient que du chlorure de sodium; et
- la poche 9 de liquide de perfusion contient une solution de chlorure de calcium, de magnésium et de potassium. Eventuellement, la poche 9 contient aussi du sodium.

L'appareil d'hémodialyse qui vient d'être décrit fonctionne de la façon suivante.

Un opérateur communique à l'unité de commande 36, par l'intermédiaire de l'interface utilisateur 37, des valeurs de consignes correspondant aux divers paramètres du traitement (prescription), à savoir, le débit du sang Qb, le débit du liquide de dialyse Qd, la perte de poids totale WL (quantité d'eau plasmatique à retirer du patient par ultrafiltration), la durée totale T de la séance, la concentration en bicarbonate [HCO3⁻]dial du liquide de dialyse (qui doit permettre à la concentration en bicarbonate du milieu intérieur du patient de tendre vers une concentration souhaitée [HCO3⁻]des), la concentration en sodium [Na⁺]dial du liquide de dialyse (qui doit permettre à la concentration en sodium du milieu intérieur du patient de tendre vers une concentration souhaitée [Na+]des), la concentration en potassium [K⁺]sol de la solution de perfusion, la concentration en potassium [K⁺]des vers laquelle la concentration du milieu intérieur du patient doit tendre. Après qu'une cartouche 18 de bicarbonate de sodium et qu'une cartouche 19 de chlorure de sodium ont été connectées aux canalisations 16, 17 correspondantes du dispositif de préparation de liquide de dialyse 14, le circuit de liquide de dialyse est rempli de liquide de dialyse. Pour ce faire, la canalisation principale 15 est raccordée à une source d'eau courante et les pompes 20, 21, 25, 26 sont mises en fonctionnement. Les pompes 20 et 21 sont réglées par l'unité de commande 36 de façon que la concentration en bicarbonate et la concentration en sodium du liquide de dialyse soient égales aux valeurs de consigne correspondantes [HCO3⁻]dial et [Na⁺]dial. Les pompes de circulation de liquide de dialyse 25, 26 sont réglées par l'unité de commande 36 de façon que le débit de la pompe 25 située en amont de l'hémodialyseur 1 soit égal au débit de consigne Qd (500 ml/min, par exemple) et que le débit de la pompe 26 située en aval de l'hémodialyseur 1 soit tel que les débits mesurés par les débitmètres 24, 27 soient égaux. Les vannes trois-voies 33, 34, 35 du dispositif de mesure de conductivité du liquide de dialyse sont agencées pour que la sonde de conductivité 32 soit normalement balayée par du liquide de dialyse frais (trajet du liquide de dialyse empruntant successivement les canalisations 12, 29, 31, 30, 12 et 13). Pour rincer et remplir initialement toutes les canalisations de liquide de dialyse frais, les vannes 33, 34, 35 sont basculées au moins une fois de façon que la sonde de conductivité 32 soit balayée par le liquide sortant de l'hémodialyseur (trajet du liquide de dialyse empruntant successivement les canalisations 12, 13, 30, 31, 29 et 13).

Simultanément au remplissage du circuit de liquide de dialyse avec le liquide de dialyse conforme à la prescription, le circuit de circulation extracorporelle de sang est rincé et rempli de liquide physiologique stérile.

Lorsque l'amorçage du circuit de liquide de dialyse et du circuit sang est achevé, le circuit sang est connecté au patient et le traitement proprement dit peut commencer : les pompes 20, 21 du dispositif 14 de préparation de liquide de dialyse, ainsi que les pompes de circulation 25, 26 du liquide de dialyse continuent de fonctionner comme pendant l'amorçage du circuit, tandis que la pompe à sang 6, la pompe d'extraction 28 et la pompe de perfusion 10 sont mises en fonctionnement. La pompe à sang 6 est réglée au débit de consigne Qb, (200 ml/mn, par exemple) et la pompe d'extraction 28 est réglée à un débit QUF calculé par l'unité de commande 36 à partir des valeurs de consigne de la perte de poids totale WL et de la durée totale du traitement T.

Conformément à l'invention, la pompe de perfusion 10 est réglée à un débit Qinf calculé par l'unité de commande 36 à partir de la formule suivante :

$$Qinf = Cl \times \frac{[K+]des}{[K+]sol - [K+]des} \qquad (1)$$

où Cl est la clairance de l'hémodialyseur 1 pour le potassium. La pompe de perfusion 10 est régulée précisément par l'unité de commande 6 à partir des informations fournies par la balance 11. La clairance Cl réelle pour le potassium est obtenue par extrapolation à partir de la dialysance réelle pour le sodium D, laquelle est déterminée par la mise en oeuvre du procédé suivant dont les étapes successives sont commandées par l'unité de commande 36. Les vannes trois voies 33, 34, 35 étant disposées de façon que le liquide de dialyse frais irrigue la sonde de conductivité 32, la conductivité Cd1in du liquide de dialyse frais correspondant à la prescription est mesurée et est mise en mémoire. Puis les trois vannes 33, 34, 35 sont basculées de façon que la sonde de conductivité 32 soit irriguée par le liquide usé et la conductivité Cd1out de ce liquide est mesurée et mise en mémoire. Le débit de la pompe 21 de solution concentrée de chlorure de sodium est alors modifié (augmenté ou diminué) de façon que la conductivité du liquide de dialyse mis en circulation soit légèrement différente de la conductivité du liquide de dialyse de la prescription. Par exemple, la conductivité du deuxième liquide de dialyse est réglée de façon à être supérieure ou inférieure de 1 mS/cm par rapport à la conductivité du premier liquide de dialyse (laquelle est généralement de l'ordre de 14 mS/cm). Comme précédemment, la conductivité Cd2in du deuxième liquide de dialyse en amont de l'hémodialyseur est mesurée et mise en mémoire, après quoi les vannes trois voies 33, 34, 35 sont basculées à nouveau pour que la sonde de conductivité 32 soit irriguée par le liquide usé, et la conductivité Cd2out du liquide usé est mesurée et mise en mémoire. La dialysance D pour le sodium peut alors être calculée par application de la formule suivante :

$$D = Qd \times \frac{(Cd1out - Cd1in) - (Cd2out - Cd2in)}{Cd2in - Cd1in} \qquad (2)$$

où Qd est le débit du liquide de dialyse.

Un autre procédé pour calculer la dialysance D à partir de mesures effectuées sur deux liquides de dialyse de conductivités différentes est décrit dans la demande de brevet EP 0 658 352.

Dans le cas particulier du traitement par hémodialyse continue, où le débit du liquide de dialyse est très inférieur au débit du sang (de l'ordre de trois fois), le débit Qinf de la solution de perfusion peut être calculé à tout moment par application de la formule :

$$Qinf = \frac{[K+]des}{[K+]sol} \times Qout \tag{3}$$

où Qout est le débit de liquide usé sortant de l'hémodialyseur, qui est égal à la somme du débit de liquide de dialyse frais, tel que déterminé par les pompes de circulation 25, 26 et du débit de la pompe d'extraction 28.

Exemple 1:

L'objectif à atteindre, en terme de concentration de potassium [K$^+$]des dans le milieu intérieur est de 2 mEq/l.

On choisit un liquide de perfusion isotonique au sang et dans lequel les proportions relatives des ions soient égales aux proportions relatives souhaitées de ces mêmes ions dans le milieu intérieur du patient. Soit, par exemple, le liquide de perfusion de composition suivante:

$$[K^+] = 42 \text{ mEq/l}$$

$$[Mg^{++}] = 31,3 \text{ mEq/l}$$

$$[Ca^{++}] = 73,5 \text{ mEq/l}$$

$$[Cl^-] = 147 \text{ mEq/l}$$

Si, la dialysance D réelle est, par exemple, de 150 ml/mn, le débit de perfusion que l'unité de commande calcule au moyen de la formule (1) et impose à la pompe de perfusion 10 est de 0,45 l/h.

Conformément à l'invention, on souhaite pouvoir utiliser l'appareil de dialyse qui vient d'être décrit pour mettre en oeuvre un traitement par hémodiafiltration avec un débit de liquide de perfusion important (supérieur à 1l/h). Dans ce cas, il n'est pas possible d'utiliser un liquide de perfusion isotonique au sang et ne comprenant que du chlorure de potassium, de calcium et de magnésium (comme celui décrit à l'exemple 1) car cela aboutirait à surcharger le patient en ces substances ioniques.

Il faut donc envisager d'utiliser un liquide de substitution dont les concentrations en potassium, calcium et magnésium soient moindres et qui contienne en outre du sodium pour rester isotonique au sang. On se heurte alors au problème de régler la concentration en sodium du liquide de dialyse en tenant compte de la perfusion, à débit variable, d'un liquide de perfusion contenant du sodium, pour que le milieu intérieur du patient tende vers une concentration souhaitée en sodium [Na+]des.

Conformément à l'invention, le débit de la pompe 21 de solution concentrée de chlorure de sodium est ajusté de façon continue pour que la concentration en sodium [Na+]dial du liquide de dialyse, telle que mesurée par la sonde de conductivité 23, soit propre à faire évoluer la concentration en sodium du milieu intérieur du patient vers une valeur souhaitée [Na+]des. La concentration en sodium [Na+]dial du liquide de dialyse est calculée par l'unité de calcul 36 en fonction de la dialysance D de l'hémodialyseur pour le sodium, du débit de perfusion Qinf, de la concentration en sodium [Na+]sol de la solution de perfusion, et de la concentration souhaitée en sodium [Na+]des vers laquelle le milieu intérieur du patient doit tendre. Elle peut être calculée par application de la formule suivante :

$$[Na+]dial = \frac{Qinf}{D} ([Na+]des - [Na+]sol) + [Na+]des \tag{4}$$

Exemple 2:

L'objectif à atteindre, en terme de concentration de potassium [K$^+$]des dans le milieu intérieur est de 2 mEq/l.

L'objectif à atteindre, en terme de concentration de sodium [Na$^+$]des dans le milieu intérieur est de 140 mEq/l.

Le débit de perfusion souhaité est de environ 1 l/h.

On choisit un liquide de perfusion isotonique au sang et dans lequel les proportions relatives des ions soient égales aux proportions relatives souhaitées de ces mêmes ions dans le milieu intérieur du patient. Soit, par exemple, le liquide de perfusion de composition suivante:

$$[K^+] = 20 \text{ mEq/l}$$

$$[Mg^{++}] = 15 \text{ mEq/l}$$

$$[Ca^{++}] = 35 \text{ mEq/l}$$

$$[Na^+] = 77 \text{ mEq/l}$$

$$[Cl^-] = 147 \text{ mEq/l}$$

Si, la dialysance D réelle est, par exemple, de 150 ml/mn, le débit de perfusion que l'unité de commande 36 calcule au moyen de la formule (1) et impose à la pompe de perfusion 10 est de 1l/h. Par ailleurs le débit que l'unité de commande 36 impose à la pompe 21 de solution concentrée de chlorure de sodium est tel que la concentration en sodium [Na+]dial du liquide de dialyse est égale à 154,8 mEq/l (concentration calculée au moyen de la formule (4)).

Exemple 3:

L'objectif à atteindre, en terme de concentration de potassium [K$^+$]des dans le milieu intérieur est de 2 mEq/l.

L'objectif à atteindre, en terme de concentration de sodium [Na$^+$]des dans le milieu intérieur est de 140 mEq/l.

Le débit de perfusion souhaité est de environ 2 l/h.

On choisit un liquide de perfusion isotonique au sang et dans lequel les proportions relatives des ions soient égales aux proportions relatives souhaitées de ces mêmes ions dans le milieu intérieur du patient. Soit, par exemple, le liquide de perfusion de composition suivante:

$$[K^+] = 10 \text{ mEq/l}$$

$$[Mg^{++}] = 7,5 \text{ mEq/l}$$

$$[Ca^{++}] = 17,5 \text{ mEq/l}$$

$$[Na^+] = 112 \text{ mEq/l}$$

$$[Cl^-] = 147 \text{ mEq/l}$$

Si, la dialysance D réelle est, par exemple, de 150 ml/mn, le débit de perfusion que l'unité de commande 36 calcule au moyen de la formule (1) et impose à la pompe de perfusion 10 est de 2,25 l/h. Par ailleurs le débit que l'unité de commande 36 impose à la pompe 21 de solution concentrée de chlorure de sodium est tel que la concentration en sodium [Na+]dial du liquide de dialyse est égale à 147 mEq/l (concentration calculée au moyen de la formule (4)).

Exemple 4:

L'objectif à atteindre, en terme de concentration de potassium [K$^+$]des dans le milieu intérieur est de 2 mEq/l.

L'objectif à atteindre, en terme de concentration de sodium [Na$^+$]des dans le milieu intérieur est de 140 mEq/l.

Le débit de perfusion souhaité est de environ 4,5 l/h.

On choisit un liquide de perfusion isotonique au sang et dans lequel les proportions relatives des ions soient égales aux proportions relatives souhaitées de ces mêmes ions dans le milieu intérieur du patient. Soit, par exemple, le liquide de perfusion de composition suivante:

$$[K^+] = 6 \text{ mEq/l}$$

$$[Mg^{++}] = 4,5 \text{ mEq/l}$$

$$[Ca^{++}] = 10,5 \text{ mEq/l}$$

$$[Na^+] = 126 \text{ mEq/l}$$

$$[Cl^-] = 147 \text{ mEq/l}$$

Si, la dialysance D réelle est, par exemple, de 150 ml/mn, le débit de perfusion que l'unité de commande 36 impose à la pompe de perfusion 10 est de 4,5 l/h. Par ailleurs le débit que l'unité de commande 36 impose à la pompe 21 de solution concentrée de chlorure de sodium est tel que la concentration en sodium [Na+]dial du liquide de dialyse est égale à 147 mEq/l (concentration calculée au moyen de la formule (4)).

Dans les exemples qui précèdent, l'objectif à atteindre a été défini en terme de concentration de potassium [K$^+$]des dans le milieu intérieur. Il va de soi que lorsque le calcium ou le magnésium sont considérés comme la substance critique pour tel ou tel patient, la pompe de perfusion 10 sera régulée en fonction d'un objectif défini en terme de concentration souhaitée de calcium [Ca$^{++}$]des ou de magnésium [Mg$^{++}$]des dans le milieu intérieur du patient.

L'invention qui vient d'être décrite est susceptible de variantes.

Dans le mode de réalisation qui a été décrit plus haut, il n'est possible de doser précisément dans le milieu intérieur qu'une des substances ioniques contenues dans le liquide de perfusion. Cela est dû au fait que la solution de perfusion utilisée est unique et contient toutes les substances ioniques à perfuser. Si l'on souhaite doser précisément plusieurs substances ioniques, il suffit d'utiliser plusieurs poches de liquide de perfusion contenant chacune une seule substance ionique à doser et de prévoir autant de moyens de perfusion (balance et pompe). On peut aussi n'utiliser qu'une seule balance, à laquelle les différentes poches sont suspendues, et qu'une seule pompe associée à des moyens de distribution pour relier tour à tour, selon une séquence temporelle déterminée, chaque poche et le circuit de circulation extracorporelle de sang.

Au lieu d'une sonde de conductivité unique baignée alternativement par le liquide de dialyse frais et le liquide usé, le circuit de liquide de dialyse peut être équipé de deux sondes de conductivité disposées sur le circuit de liquide de dialyse, respectivement en amont et en aval de l'hémodialyseur. Le circuit de liquide de dialyse peut aussi ne comporter qu'une sonde de conductivité disposée en aval de l'hémodialyseur, auquel cas les deux valeurs de consigne de conductivité utilisées pour le pilotage de la pompe de concentré 21 pour préparer le premier et le second liquides de dialyse sont substituées dans la formule indiquée ci-dessus, aux valeurs de conductivité mesurées en amont de l'hémodialyseur.

**Revendications**

1.  Appareil de dialyse comprenant:

    - des moyens (12, 13, 25, 26) pour mettre en circulation dans un hémodialyseur (1) un liquide de dialyse contenant du chlorure de sodium et du bicarbonate de sodium;
    - des moyens (9, 12) pour perfuser à un patient au moins une solution contenant au moins une substance ionique A absente du liquide de dialyse, la substance A ayant une concentration déterminée [A]sol, dans la solution de perfusion;

    **caractérisé en ce qu'**il comporte :

- des moyens (29, 30, 31, 32, 33, 34, 35, 36) pour déterminer la dialysance réelle D de l'hémodialyseur (1) pour le sodium;
- des moyens (36) pour déterminer un débit Qinf de solution de perfusion pour que la concentration de la substance A dans le milieu intérieur du patient tende vers une concentration souhaitée [A]des, en fonction de la dialysance D, de la concentration [A]sol de la substance A dans la solution de perfusion et de la concentration souhaitée [A]des;
- des moyens de réglage (10, 11) pour régler le débit de solution de perfusion;
- des moyens de commande (36) pour piloter les moyens de réglage (10, 11 ) du débit de la solution de perfusion pour que ce débit soit sensiblement égal au débit déterminé Qinf.

2. Appareil de dialyse selon la revendication 1, **caractérisé en ce que** les moyens (36) de détermination du débit de perfusion Qinf comprennent des moyens de calcul pour calculer le débit de perfusion Qinf selon la formule :

$$Qinf = Cl \times \frac{[A]des}{[A]sol - [A]des}$$

où Cl est la clairance de l'hémodialyseur (1) pour la substance A, extrapolée à partir de la dialysance D pour le sodium.

3. Appareil de dialyse selon une des revendications 1 et 2, **caractérisé en ce que** la solution de perfusion contient en outre du sodium à une concentration déterminée [Na+]sol.

4. Appareil de dialyse selon la revendication 3, **caractérisé en ce qu'**il comporte en outre:

- des moyens (14) de préparation du liquide de dialyse comprenant des moyens (21, 23) pour régler la concentration en sodium du liquide de dialyse;
- des moyens (36) pour déterminer la concentration en sodium [Na+]dial du liquide de dialyse pour que la concentration du milieu intérieur du patient tende vers une concentration souhaitée en sodium [Na+]des, en fonction de la dialysance D, du débit du liquide de perfusion Qinf, de la concentration en sodium [Na+]sol de la solution de perfusion et de la concentration en sodium souhaitée [Na+]des;
- des moyens (36) de commande pour piloter les moyens de réglage (21) de la concentration en sodium du liquide de dialyse de façon que cette concentration soit égale à la concentration déterminée [Na+]dial.

5. Appareil selon la revendication 4, **caractérisé en ce que** les moyens pour déterminer la concentration en sodium [Na+]dial du liquide de dialyse comprennent des moyens de calcul (36) pour calculer cette concentration selon la formule :

$$[Na+]dial = \frac{Qinf}{D} ([Na+]des - [Na+]sol) + [Na+]des$$

6. Appareil selon une des revendications 1 à 5, **caractérisé en ce que** les moyens pour déterminer la dialysance D de l'hémodialyseur (1) pour le sodium comprennent :

- des moyens (32) pour mesurer la conductivité du liquide de dialyse en amont et en aval de l'hémodialyseur (1) ;
- des moyens (36) pour calculer la dialysance D à partir de au moins deux valeurs de conductivité (Cd1in, Cd1out, Cd2in, Cd2out) mesurées respectivement en amont et en aval de l'hémodialyseur (1) dans au moins deux liquides de dialyse préparés successivement.

7. Appareil selon la revendication 6, **caractérisé en ce que** les moyens de calcul (36) sont prévus pour calculer D selon la formule suivante :

$$D = Qd \times \frac{(Cd1out - Cd1in) - (Cd2out - Cd2in)}{Cd2in - Cd1in}$$

où Qd est le débit de liquide de dialyse.

8. Appareil selon une des revendications 1 à 7, **caractérisé en ce qu'**il comporte en outre des moyens (14) pour préparer un liquide de dialyse à partir d'une première solution concentrée de chlorure de sodium et d'une seconde solution concentrée de bicarbonate de sodium, ces moyens de préparation comprenant des moyens (20, 21, 22, 23) pour régler indépendamment l'une de l'autre la concentration en sodium et la concentration en bicarbonate du liquide de dialyse.

9.  Appareil selon la revendication 8, **caractérisé en ce que** les moyens (14) de préparation de liquide de dialyse comprennent des moyens (19) pour produire la première solution concentrée à partir de chlorure de sodium sous forme de poudre ou de granulés.

10. Appareil selon une des revendications 8 et 9, **caractérisé en ce que** les moyens (14) de préparation de liquide de dialyse comprennent des moyens (18) pour produire la deuxième solution concentrée à partir de bicarbonate de sodium sous forme de poudre ou de granulés.

11. Appareil selon une des revendications 1 à 10, **caractérisé en ce que** les moyens de perfusion (9, 12) sont prévus pour être connectés à un circuit extracorporel de sang (5, 7) incluant le compartiment sang (2) de l'hémodialyseur (1), en aval de l'hémodialyseur.

12. Appareil selon une des revendications 1 à 11, **caractérisé en ce que** la substance A est choisie parmi le calcium, le potassium et le magnésium.

**Patentansprüche**

1.  Dialysegerät mit:

    -   Mitteln (12, 13, 25, 26), um in einem Hämodialysegerät (1) eine Dialyseflüssigkeit in Zirkulation zu versetzen, die Natriumchlorid und Natriumbikarbonat enthält;

    -   Mitteln (9, 12), um einem Patienten mindestens eine Lösung zu perfundieren, die mindestens eine ionische Substanz A enthält, die in der Dialyseflüssigkeit abwesend ist, wobei die Substanz A in der Perfusionslösung eine bestimmte Konzentration [A]sol hat,

    **dadurch gekennzeichnet, dass** es Folgendes umfasst:

    -   Mittel (29, 30, 31, 32, 33, 34, 35, 36) zum Bestimmen der tatsächlichen Dialysierfähigkeit D des Hämodialysegeräts (1) für Natrium;

    -   Mittel (36) zum Bestimmen eines Perfusionslösungsdurchsatzes Qinf, damit die Konzentration der Substanz A im inneren Milieu des Patienten in Abhängigkeit von der Dialysierfähigkeit D, der Konzentration [A]sol der Substanz A in der Perfusionslösung und von der gewünschten Konzentration [A]des zu einer gewünschten Konzentration [A]des tendiert;

    -   Einstellmittel (10, 11) zum Einstellen des Perfusionslösungsdurchsatzes;

    -   Steuermittel (36) zum Steuern der Einstellmittel (10, 11) des Durchsatzes der Perfusionslösung, damit dieser Durchsatz im Wesentlichen gleich dem bestimmten Durchsatz Qinf ist.

2.  Dialysegerät nach Anspruch 1, **dadurch gekennzeichnet, dass** die Mittel (36) zum Bestimmen des Perfusionsdurchsatzes Qinf Rechenmittel zum Berechnen des Perfusionsdurchsatzes Qinf nach der folgenden Formel umfassen:

$$Qinf = Cl \times \frac{[A]des}{[A]sol - [A]des}$$

    wobei Cl die Clearance des Hämodialysegeräts (1) für die Substanz A extrapoliert ausgehend von der Dialysierfähigkeit D für Natrium ist.

3.  Dialysegerät nach Anspruch 1 oder 2, **dadurch gekennzeichnet, dass** die Perfusionslösung außerdem Natrium mit einer bestimmten Konzentration [Na$^+$]sol enthält.

4. Dialysegerät nach Anspruch 3, **dadurch gekennzeichnet, dass** es außerdem Folgendes umfasst:

   - Mittel (14) zur Herstellung der Dialyseflüssigkeit, die Mittel (21, 23) zum Einstellen der Natriumkonzentration der Dialyseflüssigkeit umfassen;

   - Mittel (36) zum Bestimmen der Natriumkonzentration [Na$^+$]dial der Dialyseflüssigkeit, damit die Konzentration im inneren Milieu des Patienten in Abhängigkeit von der Dialysierfähigkeit D, vom Durchsatz der Perfusionsflüssigkeit Qinf, von der Natriumkonzentration [Na$^+$]sol der Perfusionslösung und der gewünschten Natriumkonzentration [Na$^+$]des zu einer gewünschten Natriumkonzentration [Na$^+$]des tendiert;

   - Steuermittel (36) zum Steuern der Einstellmittel (21) der Natriumkonzentration der Dialyseflüssigkeit so, dass diese Konzentration gleich der bestimmten Konzentration [Na$^+$]dial ist.

5. Gerät nach Anspruch 4, **dadurch gekennzeichnet, dass** die Mittel zum Bestimmen der Natriumkonzentration [Na$^+$]dial der Dialyseflüssigkeit Rechenmittel (36) umfassen, um diese Konzentration nach der folgenden Formel:

$$[Na^+]dial = \frac{Qinf}{D}([Na^+]des - [Na^+]sol) + [Na^+]des$$

   zu berechnen.

6. Gerät nach einem der Ansprüche 1 bis 5, **dadurch gekennzeichnet, dass** die Mittel zum Bestimmen der Dialysierfähigkeit D des Hämodialysegeräts (1) für Natrium Folgendes umfassen:

   - Mittel (32) zum Messen der Leitfähigkeit der Dialyseflüssigkeit stromaufwärts und stromabwärts vom Hämodialysegerät (1);

   - Mittel (36) zum Berechnen der Dialysierfähigkeit D ausgehend von mindestens zwei Leitfähigkeitswerten (Cd1in, Cd1out, Cd2in, Cd2out), die jeweils stromaufwärts und stromabwärts vom Hämodialysegerät (1) in mindestens zwei nach einander hergestellten Dialyseflüssigkeiten gemessen werden.

7. Gerät nach Anspruch 6, **dadurch gekennzeichnet, dass** die Rechenmittel (36) vorgesehen sind, um D nach der folgenden Formel zu berechnen:

$$D = Qd \; x \; \frac{(Cd1out - Cd1in) - (Cd2out - Cd2in)}{Cd2in - Cd1in}$$

   wobei Qd der Dialyseflüssigkeitsdurchsatz ist.

8. Gerät nach einem der Ansprüche 1 bis 7, **dadurch gekennzeichnet, dass** es außerdem Mittel (14) zum Herstellen einer Dialyseflüssigkeit ausgehend von einer ersten konzentrierten Natriumchloridlösung und einer zweiten konzentrierten Natriumbikarbonatlösung umfasst, wobei diese Herstellungsmittel Mittel (20, 21, 22, 23) umfassen, um unabhängig von einander die Natriumkonzentration und die Bikarbonatkonzentration der Dialyseflüssigkeit einzustellen.

9. Gerät nach Anspruch 8, **dadurch gekennzeichnet, dass** die Mittel (14) zur Herstellung der Dialyseflüssigkeit Mittel (19) zum Herstellen der ersten konzentrierten Lösung ausgehend von Natriumchlorid in Form von Pulver oder Granulat umfassen.

10. Gerät nach Anspruch 8 oder 9, **dadurch gekennzeichnet, dass** die Mittel (14) zur Herstellung der Dialyseflüssigkeit Mittel (18) zum Herstellen der zweiten konzentrierten Lösung ausgehend von Natriumbikarbonat in Form von Pulver oder Granulat umfassen.

11. Gerät nach einem der Ansprüche 1 bis 10, **dadurch gekennzeichnet, dass** die Perfusionsmittel (9, 12) vorgesehen sind, um stromabwärts vom Hämodialysegerät an einen extrakorporalen Blutkreislauf (5, 7) angeschlossen zu werden, der die Blutzelle (2) des Hämodialysegeräts (1) enthält.

12. Gerät nach einem der Ansprüche 1 bis 11, **dadurch gekennzeichnet, dass** die Substanz A aus Calcium, Kalium und Magnesium ausgewählt wird.

**Claims**

1. Dialysis apparatus comprising:

   - means (12, 13, 25, 26) for circulating a dialysis liquid containing sodium chloride and sodium bicarbonate through a haemodialyser (1 );
   - means (9, 12) for infusing a patient with at least one solution containing at least one ionic substance A absent from the dialysis liquid, the substance A having a determined concentration [A]sol in the infusion solution;

   **characterized in that** it comprises:

   - means (29, 30, 31, 32, 33, 34, 35, 36) for determining the actual dialysance D of the haemodialyser (1) for sodium;
   - means (36) for determining a flow rate Qinf of infusion solution such that the concentration of the substance A inside the patient's body tends towards a desired concentration [A]des, as a function of the dialysance D, of the concentration [A]sol of the substance A in the infusion solution, and of the desired concentration [A]des;
   - regulating means (10, 11) for regulating the flow rate of infusion solution;
   - control means (36) for driving the means (10, 11 ) for regulating the flow rate of the infusion solution such that this flow rate is substantially equal to the determined flow rate Qinf.

2. Dialysis apparatus according to Claim 1, **characterized in that** the means (36) for determining the infusion flow rate Qinf comprise calculation means for calculating the infusion flow rate Qinf according to the formula:

$$Qinf = Cl \times \frac{[A]des}{[A]sol - [A]des}$$

   where Cl is the clearance of the haemodialyser (1) for the substance A, extrapolated on the basis of the dialysance D for sodium.

3. Dialysis apparatus according to one of Claims 1 and 2, **characterized in that** the infusion solution contains moreover sodium at a determined concentration [Na+]sol.

4. Dialysis apparatus according to Claim 3, **characterized in that** it further includes:

   - means (14) for preparing the dialysis liquid, comprising means (21, 23) for regulating the sodium concentration of the dialysis liquid;
   - means (36) for determining the sodium concentration [Na+]dial of the dialysis liquid such that the concentration inside the patient's body tends towards a desired sodium concentration [Na+]des, as a function of the dialysance D, of the flow rate of the infusion liquid Qinf, of the sodium concentration [Na+]sol of the infusion solution, and of the desired sodium concentration [Na+]des;
   - control means (36) for driving the means (21) for regulating the sodium concentration of the dialysis liquid such that this concentration is equal to the determined concentration [Na+]dial.

5. Apparatus according to Claim 4, **characterized in that** the means for determining the sodium concentration [Na+]dial of the dialysis liquid comprise calculation means (36) for calculating this concentration according to the formula:

$$[Na+]dial = \frac{Q\,inf}{D}\,([Na+]des - [Na+]sol) + [Na+]des$$

6. Apparatus according to one of Claims 1 to 5, **characterized in that** the means for determining the dialysance D of the haemodialyser (1) for sodium comprise:

   - means (32) for measuring the conductivity of the dialysis liquid upstream and downstream of the haemodialyser (1);
   - means (36) for calculating the dialysance D on the basis of at least two conductivity values (Cd1in, Cd1out, Cd2in, Cd2out) measured respectively upstream and downstream of the haemodialyser (1) in at least two successively prepared dialysis liquids.

**7.** Apparatus according to Claim 6, **characterized in that** the calculation means (36) are designed to calculate D according to the following formula:

$$D = Qd \times \frac{(Cd1out - Cd1in) - (Cd2out - Cd2in)}{Cd2in - Cd1in}$$

where Qd is the flow rate of dialysis liquid.

**8.** Apparatus according to one of Claims 1 to 7, **characterized in that** it further includes means (14) for preparing a dialysis liquid from a first concentrated solution of sodium chloride and a second concentrated solution of sodium bicarbonate, these preparation means comprising means (20, 21, 22, 23) for regulating the sodium concentration and the bicarbonate concentration of the dialysis liquid independently of one another.

**9.** Apparatus according to Claim 8, **characterized in that** the means (14) for preparing dialysis liquid comprise means (19) for producing the first concentrated solution from sodium chloride in powder or granular form.

**10.** Apparatus according to one of Claims 8 and 9, **characterized in that** the means (14) for preparing dialysis liquid comprise means (18) for preparing the second concentrated solution from sodium bicarbonate in powder or granular form.

**11.** Apparatus according to one of Claims 1 to 10, **characterized in that** the infusion means (9, 12) are designed to be connected to an extracorporeal blood circuit (5, 7) which includes the blood compartment (2) of the haemodialyser (1), downstream of the haemodialyser.

**12.** Apparatus according to one of Claims 1 to 11, **characterized in that** the substance A is selected from calcium, potassium and magnesium.

UNITE
DE
COMMANDE

Eau

EP 0 898 976 B1